(19) Europäisches Patentamt
European Patent Office
Office européen des brevets

(11) EP 1 225 860 B1

(12) EUROPÄISCHE PATENTSCHRIFT

(45) Veröffentlichungstag und Bekanntmachung des Hinweises auf die Patenterteilung:
**04.06.2003 Patentblatt 2003/23**

(21) Anmeldenummer: **00958406.1**

(22) Anmeldetag: **09.08.2000**

(51) Int Cl.[7]: **A61F 13/15**, A61K 7/32, C08L 5/16, C08L 33/02

(86) Internationale Anmeldenummer:
**PCT/EP00/07741**

(87) Internationale Veröffentlichungsnummer:
**WO 01/013841 (01.03.2001 Gazette 2001/09)**

(54) **WASSERABSORBIERENDE POLYMERE MIT HOHLRAUMVERBINDUNGEN, VERFAHREN ZU DEREN HERSTELLUNG UND DEREN VERWENDUNG**

HYDROABSORBENT POLYMERS COMPRISING INTERSTITIAL COMPOUNDS, METHOD FOR PRODUCING AND USING THE SAME

POLYMERES HYDROABSORBANTS COMPORTANT DES COMPOSES A CAVITES, LEURS PROCEDES DE PRODUCTION ET LEUR UTILISATION

(84) Benannte Vertragsstaaten:
**AT BE CH CY DE DK ES FI FR GB GR IE IT LI LU MC NL PT SE**

(30) Priorität: **20.08.1999 DE 19939662**

(43) Veröffentlichungstag der Anmeldung:
**31.07.2002 Patentblatt 2002/31**

(73) Patentinhaber: **STOCKHAUSEN GmbH & Co. KG**
**47805 Krefeld (DE)**

(72) Erfinder:
- **BREHM, Helmut**
  **47800 Krefeld (DE)**
- **HARREN, Jörg**
  **47807 Krefeld (DE)**
- **ISSBERNER, Jörg**
  **47804 Krefeld (DE)**
- **MERTENS, Richard**
  **47803 Krefeld (DE)**

(74) Vertreter: **Kahlhöfer, Hermann, Dipl.-Phys. et al**
**Patentanwälte**
**Kahlhöfer Neumann**
**Herzog Fiesser**
**Karlstrasse 76**
**40210 Düsseldorf (DE)**

(56) Entgegenhaltungen:
**WO-A-94/22501** **FR-A- 2 755 612**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 225 860 B1

Printed by Jouve, 75001 PARIS (FR)

## Beschreibung

Die Erfindung betrifft Absorptionsmittel für Wasser und wäßrige Flüssigkeiten auf Basis wasserquellbarer, aber nicht wasserlöslicher Polymere, in welche Cyclodextrin oder Cyclodextrinderivate und siliziumreiche Zeolithe ionisch, kovalent und/oder aufgrund mechanischer Einschlüsse eingebunden sind.

Bei den kommerziell verfügbaren superabsorbierenden Polymeren handelt es sich im wesentlichen um vernetzte Polyacrylsäuren, vernetzte Stärke/Acrylsäure-Pfropfcopolymerisate, vernetzte hydrolysierte Stärke/Acrylnitril-Pfropfcopolymerisate, vernetztes Poly(maleinsäureanhydrid-co-isobutylen) oder Mischungen verschiedener vorgenannter vemetzter Polymere, bei denen die Carboxylgruppen teilweise mit Natrium- und/oder Kalium-lonen neutralisiert sind. Solche Polymere finden ihren Einsatz z.B. in Hygieneartikeln, die Körperflüssigkeiten wie z.B. Urin oder Menstrualflüssigkeit aufsaugen können oder in Aufsaugkissen in Verpackungen für Lebensmittel. Dort nehmen sie unter Quellung und Ausbildung von Hydrogelen große Mengen an wäßrigen Flüssigkeiten und Körperflüssigkeiten, wie z. B. Urin oder Blut auf. Ferner ist es notwendig, daß die aufgenommene Flüssigkeitsmenge unter dem anwendungstypischen Druck zurückgehalten wird. im Zuge der technischen Weiterentwicklung der superabsorbierenden Polymere hat sich das Anforderungsprofil an diese Produkte über die Jahre hinweg deutlich verändert.

Bisher wurde die Entwicklung von Superabsorbem besonders im Hinblick auf die aufgenommene Flüssigkeitsmenge und die Druckstabilität forciert.
Diese vemetzten Polymerisate auf Basis säuregruppenhaltiger Monomere werden durch die Verwendung eines oder mehrerer Vorvemetzer und/oder eines oder mehrerer Nachvemetzer erhalten und zeigen bislang nicht erreichte Eigenschaftskombinationen aus hoher Retention, hoher Absorption unter Druck, niedrigen löslichen Anteilen, schneller Flüssigkeitsaufnahme und hoher Permeabilität im gequollenem Zustand. Eingesetzt in Hygieneartikeln haben diese vemetzten Polymerisate den Vorteil, daß die ausgeschiedenen Flüssigkeiten, einmal von dem Polymerisat aufgesogen, keinen Hautkontakt mehr herstellen können. Hautschädigungen wie z.B. Windeldermatitis können so im Wesentlichen vermieden werden. Dieser Komfort kann zusätzlich gesteigert werden, indem geruchsbelästigende Verbindungen absorbiert werden.

Laut Römpp Chemie Lexikon "unterliegt der Gehalt an Ham-Inhaltsstoffen und damit an geruchsbelästigenden Verbindungen physiologischen Schwankungen; manche Substanzen werden auch in tagesperiodisch wechselnden Konzentration ausgeschieden, so daß genauere Angaben über die Zusammensetzung des Harns sich stets auf den sog. 24-Stunden-Ham beziehen. Dieser enthält beim gesunden Erwachsenen z.B. Harnstoff (durchschnittlich 20 g), Harnsäure (0,5 g), Kreatinin (1,2 g), Ammoniak (0,5 g), Aminosäuren (2 g), Proteine (60 mg), reduzierende Substanzen (0,5 g, davon etwa 70 mg D-Glucose od. Harnzucker), Zitronensäure (0,5 g) u.a. org. Säuren sowie einige Vitamine (C, $B_{12}$ u.a.). An anorganischen Ionen liegen vor: $Na^+$ (5,9 g), $K^+$ (2,7 g), $NH_4^+$ (0,8 g), $Ca^{2+}$ (0,5 g), $Mg^{2+}$ (0,4 g), $Cl^-$ (8,9 g), $PO_4^{3-}$ (4,1 g), $SO_4^{2-}$ (2,4 g). Der Trockengehalt liegt zwischen 50 u. 72 g. Als flüchtige Komponenten des Harns wurden u.a. Alkylfurane, Ketone, Lactone, Pyrrol, Allylisothiocyanat und Dimethylsulfon erkannt. Es handelt sich bei den flüchtigen Komponenten meist um Moleküle mit einer Molmasse unter ca. 1000 g/mol, die einen hohen Dampfdruck aufweisen.

Flüchtige Komponenten des Harns wurden u. a. auch von A. Zlatkis et al. (Anal. Chem. Vol. 45, 763ff.) untersucht. Bekannt ist weiterhin, daß nach Verzehr von Spargel die Konzentration von organischen schwefelhaltigen Verbindungen im Humanurin zunimmt (R. H. Waring, Xenobiotika, Vol 17, 1363ff.). Bei Patienten, die bestimmten Diäten unterliegen und/oder die bestimmte Medikamente einnehmen oder bei älteren Menschen mit nachlassender Nierenfunktion kann der Harn geruchsbelästigende Inhaltsstoffe mit sich führen. Bei Patienten mit Urin-Inkontinenz werden vermehrt Ureasen ausgeschieden, die den im Harn befindlichen Harnstoff umsetzen und so giftiges Ammoniak freisetzen. Bekannt ist ferner eine krankhafte Veränderung, die Fisch-Geruch-Syndrom genannt wird. Sie beruht auf vermehrter Ausscheidung von quartären Ammoniumverbindungen. Auch Menstrualflüssigkeit kann einen unangenehmen Geruch bekommen. Dieser Geruch ensteht u. a. durch mikrobiellen Abbau von Proteinen, die ausgeschieden werden. Die typischen Geruchsstoffe in der Menstrualflüssigkeit und die durch den Abbau von Blutinhaltsstoffen hervorgebrachten Gerüche unterscheiden sich nicht wesentlich von den Gerüchen, der im Harn vorkommenden Inhaltsstoffe. Es sind auch hier niedermolekulare Verbindungen mit einem Molgewicht von weniger als 1000 g/mol. Vorwiegend sind stickstoffhaltige Heterocyclen wie z.B. Pyrrol, Pyridin und deren Derivate zu nennen. Weiterhin sind Gerüche zu verzeichnen, die von Lebensmitteln ausgehen z.B. Fischgeruch (Amine).

Die Geruchskomponenten von vaginalen Sekretionen und Menstruationsflüssigkeit wurden von G. Huggins und G. Preti (Clinical Obstetrics and Gynecology, Vol. 24, No. 2, June 1981, 355-377) untersucht. Gefunden wurden niedrigmolekulare Substanzen mit einem Molgewicht unter 500 g/mol. Hervorzuheben sind hier Fettsäuren (z.B. Buttersäure, Isovaleriansäure) und einige aromatische Verbindungen wie z.B. Pyridin, Indol und Thymin, die besonders zum schlechten Geruch beitragen. Die Menge an flüchtigen Fettsäuren variiert über den Zeitraum des Menstruationszyklus (*Human Vaginal Secretions: Volatile Fatty Acid Content,* Richard P. Michael, R. W. Bonsall, Patricia Warner, *Science,* 27 December **1974**, 1217-1219). Amine wurden in den Vaginal-Sekretionen und Menstruationsflüssigkeiten nicht gefunden. Dies liegt daran, daß der pH Wert der Sekretion bei einer gesunden Patientin im sauren Bereich liegt

und dort allenfalls Ammoniumsalze vorliegen, die nicht flüchtig sind. Erst bei krankhaften Zuständen können durch bakteriellen Abbau vermehrt Proteine in Amine umgewandelt werden, die bei gleichzeitiger pH-Wert-Erhöhung in den Dampfraum gelangen.

Bisherige Ansätze, um z.B. bei Inkontinenzprodukten und Produkten für die Damenhygiene eine Geruchsverminderung zu erzielen, beruhen auf einer Reduzierung der freien Ammoniak-Konzentration. Dazu gibt es grundsätzlich zwei Ansätze: Verhinderung der zusätzlichen Ammoniak-Produktion aus Harnstoff Abbau durch geeignete Urease-Hemmer (A. Norberg et al. Gerontology, 1984, 30, 261ff) oder durch Protonierung von freiem Ammoniak und Bindung desselben als Ammoniumsalz von Carboxylaten. Nachteilig ist bei diesen Verfahren, daß sich im wesentlichen nur Ammoniak und andere stickstoffhaltige Komponenten kontrollieren lassen. Geruchsbelästigende Verbindungen ohne basische Gruppierungen, z.B. Thiole, werden weiterhin in den Dampfraum eintreten können.

Dem Fachmann ist bekannt, daß gewisse Hohlraum-Moleküle, auch endohedrale oder konkave Moleküle genannt, andere, meist kleinere, sogenannte Gastmoleküle aufnehmen können und somit einen Wirt/Gastkomplex bilden. Durch diese Komplexbildung werden die chemischen und physikalischen Eigenschaften des Gast- und des Wirtmoleküls beeinflußt. Cyclodextrine entstehen beim Abbau von Stärke durch Bacillus macerans oder Bacillus circulans unter Einwirkung von Cyclodextringlycosyltransferase. Sie bestehen aus 6, 7 oder 8 zu einem Zyklus $\alpha$-1,4-verknüpften Glucose-Einheiten ($\alpha$-, $\beta$- bzw. $\gamma$-Cyclodextrine). Sie sind in der Lage, hydrophobe Gastmoleküle in wechselnden Mengen bis zur Sättigung einzuschließen ("molekulare Verkapselung"), z.B. Gase, Alkohole oder Kohlenwasserstoffe. Die Anwendungen von Cyclodextrinen als Wirtmolekül werden in dem Werk von J. Szejtli (Cyclodextrin Technology, Kluwer Academic Publishers, 1988) umfassend referiert.

Es sind bereits cyclodextrinhaltige Polymere bekannt. So werden in der Patentanmeldung EP 483380 A1 cyclodextrinhaltige Polymere beschrieben, bei denen Aldehydgruppen in geschützter oder ungeschützter Form in das Cyclodextrin eingebracht werden, die dann mit nukleophilen Gruppen des Polymeren zu kovalenten Bindungen reagieren.

Aus der US 5,360,899 sind vernetzte, wasserquellbare, hydrophile Cyclodextrinperlpolymerisate bekannt, die aus Hydroxyalkylcyclodextrinen und Vemetzem vom Typ Epichlorhydrin bzw. Polyepoxiden mit nachfolgender Alkoxylierung aufgebaut werden. Diese polymerimmobilisierten Cyclodextrine werden zur Verwendung in chromatographischen Trennsäulen vorgeschlagen.

Aus der US 5,357,012 sind vemetzte, wasserquellbare, hydrophile Cyclodextrinperlpolymerisate bekannt, die aus Methacrylatgruppen tragenden Cyclodextrinen und Comonomeren wie etwa Hydroxyethylacrylat aufgebaut sind.

Auch diese polymerimmobilisierten Cyclodextrine werden zur Verwendung in chromatographischen Trennsäulen vorgeschlagen.

Die DE 195 20 989 A1 beschreibt die kovalente Anbindung von reaktiven Cyclodextrinderivaten mit mindestens einem stickstoffhaltigen Heterocyclus an Polymere, die mindestens eine nukleophile Gruppe tragen. Beispiele für nukleophile Produkte sind : -OH, -NH, oder SH-Gruppen . Auch werden polymerisierbare Cyclodextrinderivate erwähnt, die nach geeigneter Modifizierung mit anderen Monomeren z.B. ethylenisch ungesättigte Verbindungen copolymerisiert werden.

Der Einsatz von nicht-derivatisiertem Cyclodextrin als Feststoff in Hygieneprodukten wird z.B. in der EP 806 195 A1, WO 94/22500 gelehrt. Dabei werden auch Beschichtungen von pulverförmigen Absorptionsmittel beschrieben. Nachteilig ist jedoch, daß zwischen Cyclodextrin und dem pulverförmigen Absorptionsmittel keine Bindung besteht und das Cyclodextrin im Anwendungsfall ausgewaschen werden kann und es bei Lagerung oder Transport zur Entmischung kommt. Letztlich führen diese Mischungen aus Absorptionsmittel und Cyclodextrin zu einem Effektivitätsverlust bei der Geruchsbindung, da die das wäßrige Medium aufsaugenden Absorber von dem Geruchbindemittel Cyclodextrin durch Entmischungsvorgänge überwiegend getrennt vorliegen.

Um eine bessere Haftung des Cyclodextrins auf den pulverförmigen Absorptionsmitteln zu erlangen, lehrt die WO 94/22501 Cyclodextrin mit Polyethylenglykolen oder anderen linearen Polymeren in der "Schmelze" oder in Lösung zu versetzen und dann auf das pulverförmige Absorptionsmittel aufzusprühen. Dem Fachmann ist jedoch bekannt, daß sich lineare Polymere bevorzugt in den Cyclodextrin-Hohlraum "einfädeln", was man sich in der Supramolekularen Chemie vorteilhaft zu Nutze macht, um z.B. Rotaxane oder Catenane herzustellen (vgl. dazu die Schriften US 5538655, G. Wenz, Angew. Chem. 1994, 106, 851). Das in der WO 94/22501 beschriebene Verfahren ist deshalb besonders nachteilig, weil die Cyclodextrin-Hohlräume nach dieser Vorbehandlung mit einem Polyethylenglykol nicht mehr quantitativ zur Aufnahme von geruchsbelästigenden Verbindungen zu Verfügung stehen.

Den aufgeführten Druckschriften, z.B. EP 806195 A1, WO 94/22501, WO 94/22500, sind hinsichtlich einer Geruchskontrolle oder Reduzierung von geruchsbelästigenden Verbindungen keine quantitativen Angaben zu entnehmen.

Zeolithe sind meist synthetische Verbindungen bestehend aus Siliziumoxid, Aluminiumoxid und einer Anzahl an Metallionen. Ihre Zusammensetzung lautet $M_2/zO \cdot Al_2O_3 \cdot xSiO_2 \cdot yH_2O$, wobei M = ein- od. mehrwertiges Metall, H, Ammonium usw., z = Wertigkeit, x=1,8 bis ca. 12 u. y=0 bis ca. 8. Strukturell bestehen Zeolithe aus $SiO_4$- und $AlO_4$-Tetraedern, die über Sauerstoff-Brücken verknüpft sind. Dabei entsteht ein Kanalsystem aus gleichgebauten und

gleichgroßen untereinander verbundenen Hohlräumen. Beim Erhitzen geben die meisten Zeolithe ihr Wasser stetig und ohne Änderung der Kristallstruktur ab. Sie können dadurch andere Verbindungen aufnehmen und fungieren dann z.B. als Katalysatoren oder Ionenaustauscher. Zeolithe zeigen weiterhin auch eine Siebwirkung, indem sie Moleküle mit kleinerem Querschnitt als die Porenöffnungen in das Kanalsystem des Gitters aufnehmen. Größere Moleküle werden ausgeschlossen.
Zum Ausgleich der negativen Ladung der $AlO_4$-Tetraeder im Alumosilicatgerüst werden Kationen benötigt.

Die Synthese von Zeolithen wird u.a. sehr ausführlich in: Zeolite Synthesis, ACS Symposium Series 398, Eds. M. L. Ocelli und H. E. Robson (1989) Seiten 2-7 beschrieben. Die Synthese von hydrophoben Zeolithen mit einem Siliziumdioxid/Aluminiumoxid-Verhältnis des Gerüstes von >100 und einer hohen hydrothermalen Stabilität und Widerstandsfähigkeit gegenüber wäßrigen alkalischen Lösungen wird in der Patentanmeldung DE 19532500A1 offenbart. Die Zeolithe weisen eine Korngröße von deutlich unter 150 µm auf.

Die Patentschrift US 4795482 lehrt den Gebrauch von hydrophoben Zeolithen zur Unterdrückung und Vermeidung von organischen Gerüchen. Die Messung der Abnahme der Gerüche erfolgte mittels Head-Space-Gaschromatographie.

Aus den Patentanmeldungen WO 91/12029 und WO 91/12031 ist bekannt, daß die in der US 4795482 beschriebenen oder auf ähnlichem Wege hergestellten hydrophoben Zeolithe in Kombination mit Superabsorbern eingesetzt werden können. Der Zeolith ist dabei "im wesentlichen" an den Superabsorber gebunden. Die so erhaltenen Komposite finden Verwendung in Hygieneartikeln wie. z.B. Windeln oder Damenbinden. Die Mischung wird erzeugt, indem im trockenen Zustand der Superabsorber mit dem Zeolith gemischt wird. Anschließend wird Wasser zugegeben, wobei ein Verklumpen der Partikel beobachtet wurde (WO 91/12031). Nach einem Trocknungsschritt kann die Mischung in Hygieneprodukte eingebracht werden. In der Patentanmeldung WO 91/12029 wird der Zeolith zusammen mit einem Bindemittel in Wasser dispergiert und in einem Coating-Prozeß auf den Superabsorber aufgebracht. Hierbei soll mindestens 20% Zeolith, bezogen auf den Superabsorber, verwendet werden.

Diese beiden Verfahrensweisen haben den Nachteil, daß die Bindung des Zeolith-Materials an das Polymere sehr schwach ist und es schon bei geringer mechanischer Belastung des Komposits zu einer Trennung und Entmischung von Superabsorber und Zeolith kommen kann. Solche mechanischen Belastungen treten z.B. bei der Förderung eines Superabsorbers und/oder eines superabsorbierende Polymere enthaltenden, absorbierenden Artikels auf. Neben der Entmischung treten dann noch die Probleme der Handhabung sehr feiner Partikel auf. Zusätzlich muß bei einer nachträglichen Behandlung des Superabsorbers mit wässrigen Dispersionen, die ggf. noch Bindemittel enthalten, mit einer Schädigung der Superabsorberstruktur und den damit verbundenen Quelleigenschaften gerechnet werden. Der hohe Anteil nichtquellbaren Zeolithmaterials im Superabsorberkomposit limitiert das Eigenschaftsprofil zusätzlich.

Aus den Patentanmeldungen EP 0 811 387A1 und EP 0 811 390A1 ist bekannt, daß Zeolithe mit einem Siliziumdioxid/Aluminiumoxid-Verhältnis von 1-5 als Geruchsabsorber in Damenbinden verwendet werden können. Die nach der Schrift hergestellten Produkte wurden einem Praxistest unterworfen und die benutzten Produkte in einem Olfaktorischen Test-Panel mit Testkandidaten bewertet. Die in den o.g. Patentanmeldungen beschriebenen Trockenmischungen entmischen sich leicht. Darüber hinaus sind die in den Patentmeldungen gelehrten Mengen an benötigtem Zeolith sehr hoch, was sich nachteilig auf den Tragekomfort von Hygieneartikeln auswirkt.

Der vorliegenden Erfindung liegt deshalb die Aufgabe zugrunde, ein absorptionsfähiges Polymerisat für Wasser und wäßrige Flüssigkeiten zur Verfügung zu stellen, das eine Substanz aufweist, mit der geruchsbelästigende organischen Verbindungen, wie sie z.B. im Harn oder anderen Ausscheidungsflüssigkeiten des Körpers vorkommen, gebunden werden und bei dem:

- die im Anwendungsfall in den Dampfraum abgegebenen geruchsbelästigenden Stoffe deutlich reduziert werden,
- eine nahezu gleichmäßige Verteilung der geruchsbindenden Substanz in dem Absorptionsmittel vorliegt,
- eine Entmischung im Zustand vor und während der Anwendung möglichst vermieden wird,
- das Absorptionsmittel gute Retentions- und Quelleigenschaften unter Druck aufweist und
- die geruchsbindende Modifikation mit möglichst kleinen Mengen der geruchsbindenden Substanz sichergestellt wird.

Der vorliegenden Erfindung liegt ferner die Aufgabe zugrunde ein Verfahren zur Herstellung des absorptionsfähigen Polymerisates zur Verfügung zu stellen, bei dem:

- insbesondere die Probleme beim Mischen von trockenen Substanzen, die sich hinsichtlich Teilchengöße wesentlich unterscheiden, wie z.B. Granulaten und Pulvern, vermieden werden,
- kein Staub entsteht und
- ein Verklumpen der Partikel während der Herstellung vermieden wird.

Die Aufgabe wird erfindungsgemäß durch ein absorptionsfähiges Polymerisat, aufgebaut aus vemetzten monoethylenisch ungesättigten, teilneutralisierten Säuregruppen tragenden Monomeren gelöst, das sowohl α, β, γ, δ-Cyclodextrine oder deren Derivate allein oder in Mischungen als auch siliziumreiche Zeolithe ionisch, kovalent oder mechanisch an- bzw. eingebunden enthält.

Siliziumreich im Sinne der Erfindung bedeutet, daß das Siliziumdioxid/Aluminiumoxid-Verhältnis >10, vorzugsweise >20, besonders bevorzugt >50, und ganz besonders bevorzugt >100 ist. Am meisten bevorzugt ist ein Siliziumdioxid/Aluminiumoxid-Verhältnis > 500.

Vernetzt im Sinne der Erfindung bedeutet, daß das Polymere vernetzt und/oder oberflächenvemetzt ist.

Erfindungsgemäß weist das absorptionsfähige Polymerisat Cyclodextrine des Typs α, β, γ, δ sowie deren Derivate auf.

Der Grundkörper eines chemisch nicht modifizierten Cyclodextrins hat die folgende Struktur:

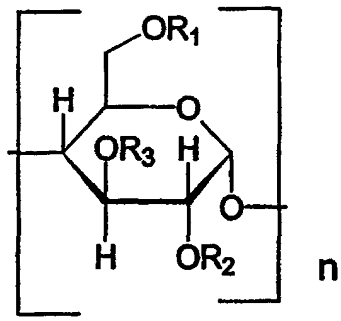

Die Anhydroglykoseeinheiten sind zyklisch zu Ringen verbunden mit $R_1$=$R_2$=$R_3$= H (α-Cyclodextrin n=6, β-Cyclodextrin: n = 7, γ-Cyclodextrin: n=8). Nicht modifizierte Cyclodextrine sind beispielsweise unter der Bezeichnung Cavitron von der Firma Cerestar oder Kleptose von der Firma Roquette oder von der Firma Wacker kommerziel erhältlich.

Bei Cyclodextrinderivaten sind pro Rest ($R_1$-$R_3$) n verschiedene Substituenten möglich, die gleich oder unabhängig voneinander chemisch unterschiedlich sein können.

Als Cyclodextrin Derivate kommen vor allem solche:in Betracht, die eine chemische Verknüpfung ionischer oder kovalenter Art mit dem Säuregruppen tragenden Absorptionsmittel ermöglichen. Kovalente Verknüpfungen werden bevorzugt über die Ausbildung von C-C-Bindungen hergestellt. Hierzu zählen beispielsweise Cyclodextrinderivate die ethylenisch ungesättigte Gruppen enthalten, die bereits bei der Polymerisation des Absorbers kovalent in die Polymerkette eingebunden werden. Solche Gruppen sind beispielsweise die (Meth)acryl-, die (Meth)allyl und die Vinylgruppe. Andererseits ist es erfindungsgemäß auch möglich die Cyclodextrine nach der Polymerisation kovalent in Form insbesondere von Ether-, Amid- oder Estergruppen an das Polymergerüst anzuknüpfen.

Die ionische Anbindung der Cyclodextrinderivate erfolgt durch anionische oder kationische Gruppen, wobei die kationischen Gruppen bevorzugt sind. Vielfach ist es von Vorteil, wenn die Cyclodextrinmoleküle mehrfach mit ionischen Gruppen substituiert sind. Beispiele für anionische Gruppen sind die Carboxylat-, die Sulfat- und die Sulfonatgruppe. Beispiele für kationische Gruppen sind die quatemierten Stickstoffe.

Die ionischen Cyclodextrine lassen sich durch Reaktion von Cyclodextrinderivaten mit reaktiven Verbindungen wie z.B. Chloressigsäure, Natriumchloracetat, Maleinsäure, Maleinsäureanhydrid, Bersteinsäureanhydrid erhalten. In wäßriger Lösung tragen derartige Umsetzungsprodukte z.B. Carboxymethylcyclodextrin im basischen eine negative Ladung durch die Carboxylat-Gruppierung.

Erfindungsgemäß zu verwendende Cyclodextrinderivate mit mindestens einem stickstoffhaltigen Heterocyclus lassen sich beispielsweise nach dem in der DE 19520989 A1 beschriebenen Verfahren herstellen. Diese Patentanmeldung wird hiermit als Referenz eingeführt und gilt somit als Teil der Offenbarung. Es lassen sich so Cyclodextrinderivate erhalten, die noch eine gegenüber nukleophilen Gruppen aktive Gruppe enthalten. Diese Derivate können direkt mit Polymeren, die ihrerseits nukleophile Gruppen tragen, reagieren. Beispiele für nukleophile Produkte sind : -OH, -NH, oder SH-Gruppen.

Weitere chemisch modifizierte, erfindungsgemäß einzusetzende Cyclodextrine lassen sich durch das in A. P. Croft und R. A. Bartsch, Tetrahedron Vol. 39, No. 9 S.1417-1473 beschriebe Verfahren herstellen. Sie werden durch Reaktion von stickstoffhaltigen Verbindungen, deren eine oder mehrere funktionelle Gruppen in der Lage sind mit Hydroxylgruppen der Cyclodextrine zu z.B. Ethem, Estern, Acetalen zu reagieren, erhalten. Diese Literaturstelle wird hiermit als Referenz eingeführt und gilt somit als Teil der Offenbarung.

Besonders bevorzugt sind kationische Cyclodextrine wie in Ch. Roussel, A. Favrou, Journal of Chromatography A, 704 (1995), 67-74 beschrieben. Sie lassen sich durch Umsetzung von Cyclodextrin mit z.B. N-(3-Chlor-2-hydroxypropyl)-N,N,N-trimethylammoniumchlorid herstellen. Diese Literaturstelle wird hiermit als Referenz eingeführt und gilt somit als Teil der Offenbarung. Die erfindungsgemäß einsetzbaren ionischen Cyclodextrine mit mindestens einem stickstoffhaltigen aliphatischen Rest lassen sich beispielsweise auch nach den in den US 3740391, 4153585 und 4638058 beschriebenen Prozeduren herstellen. Diese Patente werden hiermit als Referenz eingeführt und gelten somit als Teil der Offenbarung. Als geeignete Monomere seien beispielsweise genannt:
N,N-Dimethylaminoethyl(meth)acrylat, N,N-Dimethylaminopropyl(meth)acrylat, N,N-Dimethylaminoethyl(meth)acrylamid und N,N-Dimethylaminopropyl(meth)acrylamid. Bevorzugt Verwendung finden N,N-Dimethylaminoethylacrylat und N,N-Dimethylaminopropylacrylamid.

$$H_2C{=}CR^1\text{-}CO\text{-}X\text{-}R^2\text{-}N(R^3)_3Y^- \qquad (I)$$

in der

$R^1$ = H, $CH_3$
$R^2$ = $C_2$ - $C_4$-Alkylengruppe
$R^3$ = H, Ci - $C_4$-Alkylguppe
X = O, NH
Y = Cl, $SO_4$,

Der durchschnittliche Substitutionsgrad pro Anhydroglukose (DS-Wert von engl. degree of substitution) läßt sich für stickstoffhaltige Substituenten nach literaturbekannten Methoden über Elementaranalyse, wie beispielsweise in US 5,134,127 und US 3,453,257 für schwefel-bzw. stickstoffhaltige Substituenten beschrieben, bestimmen. Mit den in US 3,740,391, 4,153,585 beschriebenen Synthesemethoden läßt sich der DS-Wert in weiten Grenzen variieren. Diese Patente werden hiermit als Referenz eingeführt und gelten somit als Teil der Offenbarung

Pro Anhydroglykoseeinheit eines Cyclodextrins sind 3 Hydroxylgruppen zu weiteren Reaktionen befähigt. Daher kann der Substitutionsgrad z.B. im Falle des β-Cyclodextrins maximal zwischen 0,05 und 3 liegen. Ein Substitutionsgrad unter 0,05 bedeutet, daß eine Mischung aus nicht-modifiziertem Cyclodextrin und chemisch modifiziertem Cyclodextrin vorliegt.

Femer ist es möglich zur Modifizierung der Eigenschaften Cyclodextrine einzusetzen, die außer den zuvor erwähnten, für die Anbindung an den Absorber erforderlichen Gruppen, weitere, dem Polymer gegenüber nicht reaktive Substituenten enthalten. Dazu zählen beispielsweise Umsetzungsprodukte von Cyclodextrinen mit Alkylierungsmitteln, z.B. $C_1$- bis $C_{22}$-Alkylhalogeniden, z.B. Methylchlorid, Ethylchlorid, Butylchlorid, Butylbromid, Benzylchlorid, Laurylchlorid, Stearylchlorid oder Dimethylsulfat oder Umsetzungsprodukte von Cyclodextrinen mit Alkylenoxiden wie Ethylenoxid, Propylenoxid, Butylenoxid oder Styroloxid.

Die erfindungsgemäß einzusetzende Menge an Cyclodextrin oder dessen Derivat bezogen auf die Gesamtmenge pulverförmiges Absorptionsmittel beträgt 0,01-50 Gew.%, bevorzugt 0,1-30 Gew.%, besonders bevorzugt 0,5-10 Gew.%.

Die erfindungsgemäß einzusetzenden Zeolithe sind dealuminierte, hydrophobe (organophile) Zeolith-Varianten mit einem Siliziumdioxid/Aluminiumoxid-Verhältnis des Gerüstes von >10, bevorzugt >20, besonders bevorzugt >50 und ganz besonders bevorzugt >100. Am meisten bevorzugt ist ein Siliziumdioxid/Aluminiumoxid-Verhältnis > 500. Die Einsatzmenge bezogen auf die Gesamtmenge Polymerisat beträgt 0,01-10 Gew%, bevorzugt 0,1-5 Gew% und besonders bevorzugt 0,70-3 Gew%. Derartige Zeolithe werden z.B. von der Degussa AG unter dem Markennamen oder von UOP unter der Bezeichnung Abscents® in den Handel gebracht. Flavith® ist in dem Produktmerkblatt KC-CZ 42-1-05-1098 T&D genauer gekennzeichnet. Dieses Produktmerkblatt wird hiermit als Referenz eingeführt und gilt somit als Teil der Offenbarung.

Für die Polymerisation des erfindungsgemäßen Polymerisates mit ggf. superabsorbierenden Eigenschaften kommen mehrere Verfahren in Frage, z.B. Massepolymerisation, Lösungspolymerisation, Spraypolymerisation, inverse Emulsionspolymerisation und inverse Suspensionspolymerisation. Bevorzugt wird die Lösungspolymerisation in Wasser als Lösungsmittel durchgeführt. Die Lösungspolymerisation kann kontinuierlich oder diskontinuierlich erfolgen. Die Patentliteratur weist ein breites Spektrum an Variationsmöglichkeiten hinsichtlich der Konzentrationsverhältnisse, Temperaturen, Art und Menge der Initiatoren als auch der Nachkatalysatoren auf. Typische Verfahren sind in den folgenden Patentschriften beschrieben: US 4 286 082, DE 27 06 135, US 4 076 663, DE 35 03 458, DE 40 20 780, DE 42 44 548, DE 43 23 001, DE 43 33 056, DE 44 18 818. Diese Offenbarungen werden hiermit als Referenz eingeführt und gelten somit als Teil der Offenbarung

Die erfindungsgemäß einzusetzenden, säuregruppen-enthaltenden, ungesättigten Monomere sind beispielsweise Acrylsäure, Methacrylsäure, Crotonsäure, Isocrotonsäure, Maleinsäure, Fumarsäure, Itaconsäure, Vinylessigsäure, Vinylsulfonsäure, Methallylsulfonsäure, 2-Acrylamido-2-methyl-1-propansulfonsäure sowie deren Alkali- und/oder Ammoniumsalze. Bevorzugt wird Acrylsäure sowie deren Alkali- undloder Ammoniumsalze und deren Gemische verwendet. Ferner ist es auch möglich Monomere zu verwenden, die erst nach der Polymerisation zu Säuregruppen hydrolysiert werden, wie es etwa mit der Nitrilgruppe möglich ist.

Zur Modifizierung der Polymereigenschaften können optional bis zu 30 Gew.% weitere, im wäßrigen Polymerisationsansatz lösliche Comonomere wie beispielsweise Acrylamid, Methacrylamid, Acrylnitril, (Meth)allylalkoholethoxylate und die Mono(Meth)acrylsäureester von Alkoholen oder Ethoxylaten verwendet werden.

Zusammen mit den o.g. Monomeren werden in geringen Anteilen vernetzende Monomere mit mehr als einer reaktionsfähigen Gruppe im Molekül mitpolymerisiert. Dabei entstehen teilvernetzte Polymerisate, die nicht mehr in Wasser löslich sondern nur quellbar sind. Als vernetzende Monomere seien beispielsweise bi- oder mehrfunktionelle Monomere, z.B. Amide wie das Methylenbisacryl- bzw. -methacrylamid oder Ethylenbisacrylamid genannt, ferner Allylverbindungen wie Allyl(meth)acrylat, alkoxyliertes Allyl(meth)acrylat mit vorzugsweise 1 bis 30 Mol Ethylenoxid umgesetzt, Triallylcyanurat, Maleinsäurediallylester, Polyallylester, Tetraallyloxiethan, Triallylamin, Tetraallylethylendiamin, Allylester der Phosphorsäure bzw. phosphorigen Säure, ferner vemetzungsfähige Monomere, wie die N-Methylolverbindungen von Amiden wie dem Methacrylamid bzw. Acrylamid und die davon abgeleiteten Ether sowie Ester von Polyolen und alkoxylierten Polyolen, wie Diacrylate oder Triacrylate z.B. Butandiol- oder Ethylenglykoldiacrylat, Polyglykol-di-(meth)acrylate, Trimethylolpropantriacrylat, Di- und Triacrylatester des, vorzugsweise mit 1 bis 30 Mol Alkylenoxid oxalkylierten (ethoxylierten) Trimethylolpropans, Acrylat- und Methacrylatester von Glycerin und Pentaerythrit, sowie des mit vorzugsweise 1 bis 30 Mol Ethylenoxid oxethylierten Glycerins und Pentaerythrits. Bevorzugt werden Triallylamin, Acrylate mehrwertiger Alkohole bzw. deren Alkoxylate und Methallylalkoholacrylate bzw. deren Alkoxylate verwendet. Der Anteil an den vernetzenden Comonomeren liegt bei 0,01 bis 3,0 Gew.%-, bevorzugt bei 0,05 bis 2,0 Gew.% und besonders bevorzugt bei 0,05 bis 1,5 Gew.%, bezogen auf die Gesamtheit der Monomeren.

Vorzugsweise werden die sauren Monomeren neutralisiert. Die Neutralisation kann auf verschiedene Art und Weise durchgeführt werden. Zum einen kann die Polymerisation entsprechend der Lehre der US 4 654 039 direkt mit den sauren Monomeren durchgeführt werden, wobei die Neutralisation dann anschließend im Polymergel erfolgt. Diese Patentschrift wird hiermit als Referenz eingeführt und gilt somit als Teil der Offenbarung.
Zum anderen und bevorzugt werden die sauren Monomerbestandteile vor der Polymerisation zu 20-95%, bevorzugt 50-80% neutralisiert und liegen dann bereits zu Beginn der Polymerisation als Natrium-, und/oder Kalium- und/oder Ammoniumsalze vor. Zur Neutralisation werden bevorzugt solche Basen eingesetzt, die keinen negativen Einfluß auf die spätere Polymerisation haben. Bevorzugt wird Natron- und/oder Kalilauge und oder Ammoniak, besonders bevorzugt Natronlauge, eingesetzt, wobei ein Zusatz von Natriumcarbonat, Kaliumcarbonat oder Natriumbicarbonat einen zusätzlichen positiven Effekt ausüben kann, wie die US 5 314 420 und die US 5 154 713 lehren. Diese teilneutralisierte Monomerlösung wird vor dem Start der Polymerisation bei der adiabatischen Lösungspolymerisation auf eine Temperatur von unter 30°C, bevorzugt unter 20°C heruntergekühlt. Diese Patentschriften werden hiermit als Referenz eingeführt und gelten somit als Teil der Offenbarung. Bei den anderen erwähnten Verfahren sind nach dem Stand der Technik auch andere Temperaturen bekannt und üblich.

Die erfindungsgemäßen Polymerisate können wasserlösliche Polymere als Pfropfgrundlage in Mengen bis zu 40 Gew.% enthalten. Dazu zählen unter anderem teil- oder vollverseifte Polyvinylalkohole, Stärke oder Stärkederivate, Cellulose oder Cellulosederivate, Polyacrylsäuren, Polyglykole oder deren Gemische. Die Molekulargewichte der als Pfropfgrundlage zugesetzten Polymere müssen an die Gegebenheiten der Polymerisationsbedingungen angepaßt sein. So kann es z.B. im Falle einer wäßrigen Lösungspolymerisation aus Gründen der Viskosität der Polymerisatlösung erforderlich sein, nur niedrigoder mittelmolekulare Polymere einzusetzen, wohingegen bei der Suspensionspolymerisation dieser Faktor eine untergeordnete Rolle spielt.

Neben Polymerisaten, die durch vernetzende Polymerisation teilneutralisierter Acrylsäure zu erhalten sind, werden bevorzugt solche verwendet, die zusätzliche Anteile von pfropfpolymerisierter Stärke oder von Polyvinylalkohol enthalten.

Die Polymerisation kann durch verschiedene Bedingungen initiiert werden, wie z.B. durch Bestrahlung mit radioaktiven, elektromagnetischen oder ultravioletten Strahlen oder durch Redoxreaktion zweier Verbindungen, wie z. B. Natriumhydrogensulfit mit Kaliumpersulfat oder Ascorbinsäure mit Wasserstoffperoxid. Auch der thermisch ausgelöste Zerfall eines sogenannten Radikalstarters wie beispielsweise Azobisisobutyronitril, Natriumperoxidisulfat, t-Butylhydroperoxid oder Dibenzoylperoxid ist als Polymerisationsstart einsetzbar. Femer ist die Kombination von mehreren der vorgenannten Methoden möglich.

Die Herstellung des Polymerisates geschieht prinzipiell nach zwei Methoden:
Nach der ersten Methode wird die teilneutralisierte Acrylsäure in wäßriger Lösung in Gegenwart der Vernetzer und gegebenenfalls der Polymerzusätze durch radikalische Polymerisation in ein Gel überführt, das dann zerkleinert und bis zum Erreichen eines pulverförmigen, rieselfähigen Zustandes getrocknet und gemahlen und auf die gewünschte Partikelgröße abgesiebt wird. Die Lösungspolymerisation kann kontinuierlich oder diskontinuierlich durchgeführt werden. Die Patentliteratur weist sowohl ein breites Spektrum an Variationsmöglichkeiten hinsichtlich der Konzentrationsverhältnisse, Temperaturen, Art und Menge der Initiatoren als auch eine Vielzahl von Nachvemetzungsmöglichkeiten aus. Typische Verfahren sind in den folgenden Schriften beschrieben: US 4 076 663, US 4 286 082, DE 27 06 135, DE 35 03 458, DE 35 44 770, DE 40 20 780, DE 42 44 548, DE 43 23 001, DE 43 33 056, DE 44 18 818. Diese Schriften werden hiermit als Referenz eingeführt und gelten somit als Teil der Offenbarung.

Auch das inverse Suspensions- und Emulsionspolymerisationsverfahren kann zur Herstellung des Polymerisates angewendet werden. In diesen Prozessen wird eine wäßrige, teilneutralisierte Acrylsäurelösung mit Hilfe von Schutzkolloiden und/oder Emulgatoren in einem hydrophoben, organischen Lösungsmittel dispergiert und durch Radikalinitiatoren die Polymerisation gestartet Die Vemetzer sind entweder in der Monomerlösung gelöst und werden mit dieser zusammen dosiert oder aber separat und gegebenenfalls nachträglich zugefügt. Die Zugabe von gegebenenfalls vorhandenen polymeren Pfropfgrundlagen erfolgt über die Monomerlösung oder durch direkte Vorlage in die Ölphase. Anschließend wird das Wasser azeotrop aus dem Gemisch entfernt und das Polymerprodukt abfiltriert sowie optional zerkleinert und bis zum Erreichen eines pulverförmigen, rieselfähigen Zustandes getrocknet und gemahlen und auf die gewünschte Partikelgröße abgesiebt.

Die erfindungsgemäßen Polymerisate werden ggf. durch das Verfahren der nachträglichen Oberflächenvernetung in ihrem Eigenschaftsprofil verbessert, insbesondere auch in ihrer Flüssigkeitsaufnahme unter Druck, damit das bekannte Phänomen des "gel blocking" unterdrückt wird, bei dem angequollene Polymerteilchen miteinander verkleben und eine weitere Flüssigkeitsaufnahme und Flüssigkeitsverteilung z.B. in der Windel behindern. Während der Nachvemetzung werden die Carboxylgruppen der Polymermoleküle an der Oberfläche der Superabsorberteilchen mit Vernetzungsmitteln unter erhöhter Temperatur vernetzt. Verfahren zur Nachvernetzung sind in mehreren Schriften, wie z.B. der DE 40 20 780 ,der EP 317 106 und der WO 94/9043 beschrieben.

Die dem Fachmann z.B. aus der US 5 314 420, Seite 8, Zeile 3-45 bekannten Nachvemetzungsmittel können erfindungsgemäß alle vorteilhaft in Kombination mit einem Vorvemetzer oder einer Kombination von Vemetzem eingesetzt werden. Die o.g. Schriften werden hiermit als Referenz eingeführt und gelten somit als Teil der Offenbarung. Die Verbindungen enthalten in der Regel mindestens zwei funktionelle Gruppen, die zur Reaktion mit Carbonsäure- oder Carboxylgruppen befähigt sind. Dabei sind Alkohol-, Amin-, Aldehyd- und Carbonatgruppen bevorzugt, wobei auch Vemetzermoleküle mit mehreren verschiedenen Funktionen eingesetzt werden. Bevorzugt werden Polyole, Polyamine, Polyaminoalkohole, Polyepoxide und Alkylencarbonate eingesetzt. Insbesondere wird eines der folgenden Nachvernetzungsmittel eingesetzt: Ethylenglykol, Diethylenglykol, Triethylenglykol, Polyethylenglykol, Glycerin, Polyglycerin, Propylenglykol, Diethanolamin, Triethanolamin, Polypropylenglykol, Blockcopolymere aus Ethylenoxyd und Propylenoxyd, Sorbitanfettsäureester, ethoxylierte Sorbitanfettsäureester, Trimethylolpropan, ethoxyliertes Trimethylolpropan. Pentaerythrit, ethoxyliertes Pantaerythrit, Polyvinylalkol, Sorbit, Ethylencarbonat, Proypylencarbonat. Besonders bevorzugt wird mit Polyolen und Ethylencarbonat als Nachvernetzungsmittel gearbeitet. Das Nachvemetzungsmittel wird in einer Menge von 0,01 bis 30 Gewichtsprozent, bevorzugt 0,1-10 Gewichtsprozent, bezogen auf das nachzuvernetzende Polymer eingesetzt.

Vor der Nachvernetzung wird das Polymere vorzugsweise getrocknet, gemahlen und auf die für die jeweils anwendungstechnisch günstige Kornfraktion abgesiebt und dann der Nachvemetzungsreaktion zugeführt. In manchen Fällen hat es sich jedoch auch bewährt, die Nachvemetzer bereits vor der Trocknung des Polymergels bzw. vor der Zerkleinerung des teilweise oder überwiegend getrockneten Polymers zuzufügen. Eine erfindungsgemäß durchzuführende Nachvernetzung wird z.B. in der US 4 666 983 und der DE 40 20 780 beschrieben. Diese Schriften werden hiermit als Referenz eingeführt und gelten somit als Teil der Offenbarung. Der Zusatz der Nachvemetzer erfolgt häufig vorteilhafterweise auch in Form einer Lösung in Wasser, organischen Lösemitteln oder deren Mischungen, insbesondere dann, wenn geringe Mengen an Nachvernetzungsmittel angewandt werden. Geeignete Mischaggregate zum Aufbringen des Nachvernetzungsmittels sind z.B. Patterson-Kelley-Mischer, DRAIS-Turbulenzmischer, Lödigemischer, Ruberg-Mischer, Schneckenmischer, Tellermischer und Wirbelschichtmischer, sowie kontinuierlich arbeitende senkrechte Mischer in denen das Pulver mittels rotierender Messer in schneller Frequenz gemischt wird (Schugi-Mischer). Nachdem der Nachvemetzer mit dem vorvemetzten Polymer vermischt worden ist, wird zur Durchführung der Nachvemetzungsreaktion auf Temperaturen von 60 bis 250 °C, bevorzugt auf 135 bis 200°C und besonders bevorzugt auf 150 bis 185°C erhitzt. Die Zeitdauer der Nacherhitzung ist durch den Punkt begrenzt, bei dem das gewünschte Eigenschaftsprofil des Polymerisates infolge von Hitzeschädigung wieder zerstört wird.

Für die Verarbeitung der Polymerisate werden je nach Anwendungsfall unterschiedliche Siebfraktionen eingesetzt, so z.B. für Windeln zwischen 100 und 1000 µm, bevorzugt zwischen 150 und 850 µm. Diese Kornfraktion wird im allgemeinen durch Mahlung und Siebung vor und/oder nach der Nachvemetzung hergestellt.

Bei dem erfindungsgemäßen absorptionsfähigen Polymerisat für die Aufnahme von Wasser oder wässrigen Flüssigkeiten ist die Cyclodextrinkomponente undloder die Zeolithkomponenete durch die aufzusaugende Flüssigkeit nur noch in vermindertem Umfang extrahierbar bzw. im trockenen Zustand nur noch vermindert entmischbar. Überraschenderweise hat sich herausgestellt, daß die Cyclodextrine und Zeolithe durch die enge Verknüpfung mit dem vernetzten, Säuregruppen tragenden Polymerisat ihre Fähigkeit zur Geruchsbindung auch nicht teilweise einbüßen, sondern daß die Geruchsbindung im Vergleich zu ungebundenem Cyclodextrin noch verstärkt wird. Dadurch entsteht eine

effektive Verringerung der Dampfraum-Konzentration von geruchsbelästigenden Stoffen. Die geruchsbindenden Sustanzen werden z.B. aus einer wässrigen Lösung bzw. Suspension aufgebracht. Dadurch wird jegliches Staubproblem bei der Herstellung vermieden. Es hat sich weiterhin gezeigt, daß auch die Zeolithe ihre Fähigkeit zur Geruchsaufnahme nicht verlieren, wenn sie aus einer wässrigen Suspension aufgetragen werden. Es hat sich ferner gezeigt, daß die übrigen Qualitätskriterien, die für Superabsorber relevant sind, nämlich hohe Retention und Absorbtion gegen einen Druck durch die Aufbringung von den geruchsbindenden Substanzen, Cyclodextrin und Zeolith nicht nachteilig beeinflußt werden.

Dadurch, daß die Cyclodextrine und die Zeolithe in das Polymerisat eingebettet sind, entsteht eine starke Bindung zwischen dem Cyclodextrin bzw. dem Zeolith und dem Polymerisat.

Das erfindungsgemäße Polymerisat ist hervorragend zur Einlagerung von Wirkstoffen geeignet, wobei diese Wirkstoffe im Anwendungsfall gegebenenfalls wieder kontrolliert abgegeben werden können. Die Haltbarkeit empfindlicher Wirkstoffe wird durch die Einlagerung in die erfindungsgemäßen Absorptionsmittel deutlich verbessert.

Ein weiterer Gegenstand der vorliegenden Erfindung ist ein Verfahren zur Herstellung der erfindungsgemäßen absorptionsfähigen Polymerisate.

Gemäß diesem erfindungsgemäßen Verfahren erfolgt die Herstellung des erfindungsgemäßen absorptionsfähigen Polymerisates durch:

- radikalische Polymerisation einer wäßrigen Lösung aus ethylenisch ungesättigten Säuregruppen tragenden, gegebenenfalls teilneutralisierten Monomeren und vernetzenden Monomeren nach dem Verfahren der Lösungs- oder Suspensionspolymerisation zu einem Hydrogel,
- gegebenenfalls Isolierung,
- Zerkleinerung gefolgt von Trocknung, Mahlung,
- gegebenenfalls Siebung und
- Oberflächenvemetzung,

wobei die Cyclodextrinkomponente und der siliziumreiche Zeolith spätestens während der Oberflächenvemetzung des Polymerisates diesem zugesetzt werden.

Vorzugsweise erfolgt der Zusatz der Cyclodextrinkomponente und des siliziumreichen Zeoliths in Lösung bzw. in Suspension.

Siliziumreich im Sinne der Erfindung bedeutet, daß das Siliziumdioxid/Aluminiumoxid-Verhältnis >10, vorzugsweise >20, besonders bevorzugt >50, und ganz besonders bevorzugt >100 ist. Am meisten bevorzugt ist ein Siliziumdioxid/Aluminiumoxid-Verhältnis > 500.

Das Cyclodextrin oder dessen Derivat und das Zeolith werden in einem Lösungsmittel gelöst bzw. suspendiert angewendet. Ein bevorzugtes Lösemittel ist Wasser, jedoch kommen auch Gemische aus Wasser und organischen Lösemitteln zum Einsatz.

Die Zugabe des Cyclodextrins und/oder des Zeolithes kann, wie unten dargestellt, erfindungsgemäß in verschiedenen Verfahrensstufen der Herstellung des pulverförmigen Polymerisates erfolgen. Durch das Aufbringen des Cyclodextrins oder des Zeoliths aus einer wässrigen Lösung und/oder Suspension auf das Polymerisat vor oder während einem der Verfahrensschritte zu dessen Herstellung wird eine besonders effektive Bindung zwischen geruchabsorbierender Komponente und Polymerisat erreicht. Vorzugsweise wird die Lösung mit Kaliumhydroxid oder Natriumhydroxid auf einen pH-Wert von >8, vorzugsweise >9, besonders bevorzugt >11 eingestellt. Ebenfalls bevorzugt beträgt die Temperatur der Lösung 20-50°C.

Das Cyclodextrin oder dessen Derivat und Zeolith werden in einer bevorzugten Ausführungsform des erfindungsgemäßen Verfahrens direkt in die wäßrige Monomerlösung vor der Polymerisation zugegeben. Wird das absorbierende Polymerisat durch Suspensionspolymerisation hergestellt, so ist es auch möglich, das Cyclodextrin und den Zeolith in der Ölphase ganz oder teilweise vorzulegen und die Monomerlösung dazu zu dosieren. Wird nur ein Teil des Cyclodextrins und/oder des Zeoliths vorgelegt, so ist der Rest zu dosieren beispielsweise über die Monomerlösung.

In einer weiteren bevorzugten Ausführungsform des erfindungsgemäßen Verfahrens wird Cyclodextrin oder dessen Derivat und der Zeolith gelöst bzw. suspendiert in Wasser oder einem organischen Lösungsmittel oder deren Mischungen auf das zerkleinerte Polymergel aufgebracht.

Weiterhin bevorzugt wird das Polymergel zumindest teilweise getrocknet und anschließend Cyclodextrin oder dessen Derivat und Zeolith gelöst bzw. suspendiert in Wasser oder einem organischen Lösungsmittel oder deren Mischungen auf das Pulver aufgetragen. Das resultierende Produkt kann als solches direkt getrocknet und oberflächenvernetzt werden.

In einer anderen bevorzugten Ausführungsform des erfindungsgemäßen Verfahrens wird das Cyclodextrin oder dessen Derivat und Zeolith in dem Verarbeitungsschritt der Nachvernetzung eingesetzt Geeignete Mischaggregate zum Aufbringen des Nachvernetzungsmittels sind z.B. Patterson-Kelley-Mischer, DRAIS-Turbulenzmischer, Lödigemischer, Ruberg-Mischer, Schneckenmischer, Tellermischer und Wirbelschichtmischer, sowie kontinuierlich arbeitende senkrechte Mischer in denen das Pulver mittels rotierender Messer in schneller Frequenz gemischt wird (Schugi-Mischer).

Ebenfalls bevorzugt werden Cyclodextrine bzw. deren Derivate und/oder Zeolithe an mehreren Stellen des Herstellungsprozesses der absorbierenden Polymere eingebracht, um die Wirkung des Cyclodextrins und des Zeoliths zu optimieren und Synergien zu nutzen. Auf diese Art ist es beispielsweise möglich, ein nicht modifiziertes Cyclodextrin durch die Monomerlösung in das Polymerisatteilchen einzubringen und im Zuge der Oberflächennachvernetzung ein ionisch modifiziertes Cyclodextrin an der Oberfläche des Absorbers zu fixieren. Weiterhin ist es auch möglich Cyclodextrine oder deren Derivate in einem Herstellungsschritt zuzugeben und in einem anderen Herstellungsschritt den Zeolithen.

Gemäß einem weiteren erfindungsgemäßen Verfahren erfolgt die Herstellung des erfindungsgemäßen absorptionsfähigen Polymerisates durch:

- radikalische Polymerisation einer wäßrigen Lösung aus ethylenisch ungesättigten Säuregruppen tragenden, gegebenenfalls teilneutralisierten Monomeren und vernetzenden Monomeren nach dem Verfahren der Lösungs- oder Suspensionspolymerisation zu einem Hydrogel,
- gegebenenfalls Isolierung,
- Zerkleinerung gefolgt von Trocknung, Mahlung,
- gegebenenfalls Siebung,

wobei das Cyclodextrin und der siliziumreiche Zeolith dem Polymerisat zugesetzt werden, wenn dieses noch mindestens einen Wasserassergehalt von 10 Gew.% aufweist.

Vorzugsweise erfolgt der Zusatz der Cyclodextrinkomponente und des siliziumreichen Zeoliths in Lösung bzw. in Suspension.

Der Wassergehalt darf erfindungsgemäß vor der Zugabe des Cyclodextrins und des siliziumreichen Zeoliths nicht unter 10 Gew.% herabgesenkt worden sein, bevor das Cyclodextrin und der siliziumreiche Zeolith zugesetzt werden.

Vorzugsweise sollte der Wassergehalt vor der Zugabe des Cyclodextrins und des siliziumreichen Zeoliths nicht unter 30 Gew.%, besonders bevorzugt nicht unter 50 Gew.% und ganz besonders bevorzugt nicht unter 65 Gew. % herabgesenkt worden sein.

Die Zugabe des Cyclodextrins und/oder des Zeolithes erfolgt vorzugsweise aus einer wässrigen Lösung und/oder Suspension auf das Polymerisat. Vorzugsweise wird die Lösung mit Kaliumhydroxid oder Natriumhydroxid auf einen pH-Wert von >8, bevorzugt >9 und besonders bevorzugt >11 eingestellt.
Ebenfalls bevorzugt beträgt die Temperatur der Lösung 20-50°C.

Durch die erfindungsgemäßen Verfahren werden absorptionsfähige Polymecisate erhalten, in denen das Cyclodextrin oder dessen Derivat und der Zeolith im synthetischen Polymer so eingebunden ist, daß zum einen die mit Wasser extrahierbare Menge an Cyclodextrin deutlich geringer ist als die tatsächlich im Endprodukt enthaltene Menge. Der extrahierbare Anteil an Cyclodextrinen unterschreitet 85% der theoretisch im Produkt vorhandenen Menge und liegt in der Regel zwischen 45 und 60%. Der Zeolith ist auch nach mechanischer Belastung z.B. in einer Kugelmühle bei 95 UPM und 6 min nicht mehr vollständig vom Polymerisat abzutrennen. Bei dem gemäß dem erfindungsgemäßen Verfahren hergestellten Produkten lösen sich weniger als 80% in der Regel 40-60% der als Gesamtmenge auf das Polymerisat aufgebrachten Zeoliths nach einer solchen Belastung wieder ab.

Die erfindungsgemäßen Polymerisate weisen im Vergleich zu pulverförmigen Absorptionsmitteln ohne Cyclodextrin oder dessen Derivat und ohne Zeolith eine bessere Aufnahme von geruchsbelästigenden Verbindungen auf.

Das erfindungsgemäße Polymerisat findet seinen Einsatz z.B. in Hygieneartikeln, die Körperflüssigkeiten wie z.B. Urin aufsaugen können oder im Verpackungsbereich von z.B. Fleisch und Fischprodukten. Dort nehmen sie unter Quellung und Ausbildung von Hydrogelen große Mengen an wäßrigen Flüssigkeiten und Körperflüssigkeiten, wie z. B. Urin, Blut oder Fleischsaft auf. Diese Verwendungen sind deshalb ebenfalls Gegenstand der vorliegenden Erfindung.

Die erfindungsgemäßen Polymerisate werden direkt als Pulver in die Konstruktionen zur Aufnahme von Flüssigkeiten eingearbeitet oder vorher in geschäumten oder nicht geschäumten Flächengebilden fixiert. Derartige Konstruktionen zur Aufnahme von Flüssigkeiten sind beispielsweise Baby Windeln, Inkontinenzprodukte oder saugfähige Einlagen in Verpackungseinheiten für Lebensmittel. Die Bindung des Cyclodextrins oder dessen Derivates und des Zeoliths an das Polymerisat ist bei den erfindungsgemäßen absorbierenden Polymerisat offensichtlich so stark, daß auch bei mechanischer Belastung z.B. Förderung des absorbierenden Polymerisates keine wesentliche Trennung und Entmischung von Polymerisat und Zeolith beobachtbar ist und daher insbesondere die Probleme der Handhabung sehr feiner Partikel nicht auftauchen.

Vorteilhaft ist die weitere Verarbeitung des erfindungsgemäßen Polymerisates, da nach dem Stand der Technik eine getrennte gleichmäßige Dosierung von Superabsorber und Zeolith insbesondere bei kleinen Mengen Zeolith nicht erreicht werden kann. Das einfach dosierbare erfindungsgemäße Polymerisat gewährleistet eine gleichbleibende

Konzentration von Polymerisat mit superabsorbierenden Eigenschaften und geruchsbindender Komponente in absorbierenden Artikeln wie z.B. Damenbinden.
Darüber hinaus hat es sich gezeigt, daß die erfindungsgemäßen Absorptionsmittel hervorragend zur Einlagerung von Wirkstoffen geeignet sind. Die Haltbarkeit empfindlicher Wirkstoffe, beispielsweise gegen oxidativen Abbau, wird durch die Einlagerung in die erfindungsgemäßen Absorptionsmittel wesentlich verbessert.
Weiterhin findet das erfindungsgemäße Polymerisat Anwendung bei der Pflanzenaufzucht und bei der Schädlingsbekämpfung in der Landwirtschaft. In der Pflanzenaufzucht sorgen die Polymerisate in der Nähe von Pflanzenwurzeln für eine ausreichende Zufuhr von Flüssigkeit und von zuvor eingelagerten Nährstoffen und sind in der Lage diese über einen längeren Zeitraum zu speichern und wieder freizusetzen.

Bei der Schädlingsbekämpfung lassen sich im Polymerisat Wirkstoffe einzeln oder in Kombination mehrerer Wirkstoffe einlagern, die dann im Anwendungsfall zeit- und mengenkontrolliert freigesetzt werden.

In den folgenden Beispielen wird die Erfindung erläutert. Diese Erläuterungen sind lediglich beispielhaft und schränken den allgemeinen Erfindungsgedanken nicht ein.

Es werden die Herstellung und die Eigenschaften der erfindungsgemäßen Polymerisate erläutert. Femer werden die Prüfmethoden und die Vorschriften zur Bestimmung der Eigenschaften der Polymere mit superabsorbierenden Eigenschaften beschrieben.

**Prüfmethoden:**

**Prüfmethode 1:** Die Retention wird nach der Teebeutelmethode und als Mittelwert aus drei Messungen angegeben. Etwa 200 mg Polymerisat werden in einen Teebeutel eingeschweißt und für 30 Minuten in 0,9%ige NaCI-Lösung getaucht. Anschließend wird der Teebeutel in einer Schleuder (23 cm Durchmesser, 1.400 Upm) 3 Minuten geschleudert und gewogen. Einen Teebeutel ohne wasserabsorbierendes Polymerisat läßt man als Blindwert mitlaufen.

$$\text{Retention} = \text{Auswaage-Blindwert/Einwaage [g/g]}$$

**Prüfmethode 2:** Flüssigkeitsaufnahme unter Druck (AAP-Test, EP 0 339 461)

Die Aufnahme unter Druck (Druckbelastung 50 g/cm$^2$) wird nach einer in der EP 0 339 461, Seite 7, beschriebenen Methode bestimmt. Diese Schrift wird hiermit als Referenz eingeführt und ist somit Teil der Offenbarung. In einen Zylinder mit Siebboden werden ca. 0,9 g Superabsorber eingewogen. Die gleichmäßig aufgestreute Superabsorberlage wird mit einem Stempel belastet, der einen Druck von 50 g/cm$^2$ ausübt. Der zuvor gewogene Zylinder wird anschließend auf eine Glasfilterplatte gestellt, die sich in einer Schale mit 0,9%iger NaCI-Lösung befindet, deren Flüssigkeitniveau genau der Höhe der Filterplatte entspricht. Nachdem man die Zylindereinheit 1 Stunde lang 0,9%ige NaCl-Lösung saugen gelassen hat, wird diese zurückgewogen und der AAP wie folgt berechnet:

AAP = Auswaage (Zylindereinheit + Superabsorber)-Einwaage (Zylindereinheit +

vollgesogener Superabsorber) / Einwaage Superabsorber

**Prüfmethode 3)** 1 g pulverförmiges Absorptionsmittel wird mit 180 ml einer wässrigen Kochsalzlösung übergossen und 1h (alternativ 16h) bei Raumtemperatur gut gerührt. Anschließend wird durch ein Sieb filtriert und die Konzentration an Cyclodextrin gemäß nachfolgeneder Methode bestimmt.
Die Methode beruht auf der Abnahme der Licht-Absorption (550 nm) einer alkalischen Phenolphthaleinlösung in Gegenwart von Cyclodextrin und kann wie von T. Takeuchi und T. Miwa, Chromatographia 1994, 38, 453, beschrieben, bestimmt werden.
Es wird die experimentell erhaltene Konzentration durch die theoretisch berechnete Konzentration dividiert. Die theoretische Konzentration läßt sich ermitteln aus der eingesetzten Menge Cyclodextrin in dem pulverförmigen Absorptionsmittel dividiert durch 180.
Man erhält so den extrahierten Anteil an Cyclodextrin.

$$\text{EA(CD)\%} = \frac{\text{gefundene Konzentration (CD)* 100}}{\text{theoretische Konzentration (CD)}}$$

EA(CD) Extrahierbarer Anteil von Cyclodextrin

**Prüfmethode 4)** Bestimmung der Aufnahme von geruchsbelästigenden Verbindungen
0,1g pulverförmiges Absorptionsmittel werden mit 2 ml einer wässrigen Lösung (enthält 5 Gew.% Ethanol) der geruchsbelästigenden Verbindung versetzt und in einem 5 ml-fassenden Probengefäß verschlossen. Man läßt bei 23°C für 12h stehen und untersucht per Head-Space GC quantitativ den Gehalt der geruchsbelästigenden Verbindung im Dampfraum über der Flüssigkeit gegen eine Nullprobe.

**Prüfmethode 5)** Der Silizium-Gehalt der absorbierenden Polymere wird durch Umsetzung des Silicats zum Molybdänblau und anschließender photometrischer Analyse bestimmt. Silizium wird vorher durch alkalischen Aufschluß quantitativ in Silikat überführt. (Photometrische Analyse, Autoren: B. Lange, Zdenek, J. Vejdelek, S., Ausgabe 1987, S. 383, VCH)

**Beispiele:**

**Vergleichsbeispiel 1) nach Patentanmeldung WO 94/22500 und WO 94/22501**

9,850 g eines handelsüblichen pulverförmigen Absorptionsmittels (Favor®, Firma Stockhausen GmbH) werden mit 0,15 g festem β-Cyclodextrin (beta-W7-Cyclodextrin technisch, der Firma Wacker) gut durchmischt. Anschließend wird nach Prüfmethode 1) der extrahierbare Anteil an Cyclodextrin bestimmt.
EA(CD) = 93%

Bei einer Blindprobe ohne Cyclodextrin wurde kein Cyclodextrin detektiert.

**Vergleichsbeispiel 2) nach Patentanmeldung WO 94/22500 und WO 94/22501**

40 g Polyethylenglykol (Mw 3000) werden bei erhöhter Temperatur geschmolzen. Hierzu gibt man 40 g Cyclodextrin und homogenisiert die Mischung. Nach kurzer Zeit entsteht eine klare Lösung. 9,40 g eines handelsüblichen pulverförmigen Absorptionsmittels (Favor®, Firma Stockhausen GmbH) werden mit 0,6 g der Cyclodextrin/Polyethenlenglycol-Lösung besprüht, gut durchmischt und auf Raumtemperatur gekühlt. Anschließend wird nach Prüfmethode 1) der extrahierbare Anteil an Cyclodextrin bestimmt.
EA(CD) = 89 %

**Beispiel 1a)**

Mit diesem Beispiel wird die Herstellung eines Polymerisatgels mit superabsorbierenden Eigenschaften erläutert.

Es wird eine Lösung aus 1300 g Acrylsäure, 2115,9 g dest. Wasser, 2,7 g Polyethylenglycolmonoallyetheracrylat und 1,25 g Polyethylenglycoldiacrylat angesetzt. Unter Rühren und Kühlen wird mit 899,10 g 50%-iger Natronlauge teilneutralisiert (Neutralisationsgrad (NG) = 60%). Die Lösung wird auf 7-8°C abgekühlt und mit Stickstoff für ca. 20 min. durchperlt. Anschließend gibt man 0,45 g Azo-bis(2-amodinopropan)dihydrochlorid gelöst in 22,5 g dest Wasser, 1,35 g Natriumperoxodisulfat, gelöst in 25 g dest. Wasser, und 0,315 g Wasserstoffperoxid (35%-ig) gelöst in 22,5 g dest. Wasser hinzu. Danach wird die Polymerisation durch Zugabe von 0,0675 g Ascorbinsäure gelöst in 9 g Wasser gestartet, worauf eine deutliche Temperaturerhöhung stattfindet. Man erhält ein gelförmiges Produkt, dessen Weiterverarbeitung in den nachfolgenden Beispielen beschrieben wird.

**Beispiel 1b)**

Mit diesem Beispiel wird die Herstellung eines weiteren Polymerisatgels mit superabsorbierenden Eigenschaften erläutert.

Es wird eine Lösung aus 1300 g Acrylsäure, 2015,9 g dest. Wasser, 6,5 g Polyethylenglycolmonoallyetheracrylat und 3,9 g Polyethylenglycoldiacrylat angesetzt. Unter Rühren und Kühlen wird mit 997,10 g 50%-iger Natronlauge teilneutralisiert (NG = 70%). Die Lösung wird auf 7-8°C abgekühlt und mit Stickstoff für ca. 20 min. durchperlt. Anschließend gibt man 0,45 g Azo-bis(2-amidinopropan)dihydrochlorid gelöst in 22,5 g dest. Wasser,1,35 g Natriumperoxodisulfat, gelöst in 25 g dest. Wasser, und 0,315 g Wasserstoffperoxid (35%-ig) gelöst in 22,5 g dest. Wasser hinzu. Danach wird die Polymerisation durch Zugabe von 0,0675 g Ascorbinsäure gelöst in 9 g Wasser gestartet, worauf eine deutliche Temperaturerhöhung stattfindet. Man erhält ein gelförmiges Produkt, dessen Weiterverarbeitung in den nachfolgenden Beispielen beschrieben wird.

**Beispiel 1c)**

Mit diesem Beispiel wird die Herstellung eines weiteren Polymerisatgels mit superabsorbierenden Eigenschaften erläutert.

Es wird eine Lösung aus 1300 g Acrylsäure, 2017,19 g dest. Wasser, 3,9 g Triallylamin als Vernetzer angesetzt. Unter Rühren und Kühlen wird mit 997,10 g 50%-iger Natronlauge teilneutralisiert (NG = 70%). Die Lösung wird auf 7-8°C abgekühlt und mit Stickstoff für ca. 20 min. durchperlt. Anschließend gibt man 0,45 g Azo-bis(2-amidinopropan)-dihydrochlorid gelöst in 22,5 g dest. Wasser, 1,35 g Natriumperoxodisulfat, gelöst in 25 g dest. Wasser, und 0,315 g Wasserstoffperoxid (35%-ig) gelöst in 22,5 g dest. Wasser hinzu. Danach wird die Polymerisation durch Zugabe von 0,0675 g Ascorbinsäure gelöst in 9 g Wasser gestartet, worauf eine deutliche Temperaturerhöhung stattfindet. Man erhält ein gelförmiges Produkt, dessen Weiterverarbeitung in den nachfolgenden Beispielen beschreiben wird.

**Beispiele 2)**

500 g der aus Beispiel 1a) erhaltenen Gele werden gewölft mit der in der nachfolgenden Tabelle aufgeführten Mengen (Angabe in %Trockensubstanz bezogen auf Acrylsäure) an beta-Cyclodextrin, einer Suspension von Flavith® S 108 (Degussa AG, $SiO_2/Al_2O_3$ Verhältnis ca. 500) in Wasser gleichmäßig besprüht und anschließend bis zu einem Restwassergehalt von <10% bei 150°C in einem Umlufttrockenschrank getrocknet.

| Beispiel Bezeichnung. | Retention [g/g] | Beta- Cyclodextrin Gew% | Flavith S108 Gew% |
|---|---|---|---|
| 2a | 36,8 | 1 | 0,75 |
| 2b | 36,8 | 2 | 0,75 |
| 2c | 35,5 | 3 | 0,75 |
| 2d | 37,5 | 4 | 0,75 |

**Beispiel 3)**

Bei diesem Beispiel werden die Retention und die Flüssigkeitsaufnahme unter Druck eines oberflächenvernetzten Polymerisats mit superabsorbierenden Eigenschaften in Abwesenheit von Cyclodextrin oder Cyclodextrinderivaten und Zeolith untersucht.

50 g des getrockneten, gemahlenen und auf 150-850 µm abgesiebten Polymeren aus den Beispielen 1a-c) werden unter kräftigem Durchmischen mit einer Lösung aus 0,5 g Ethylencarbonat und 1,5 g Wasser in einem Kunststoffgefäß benetzt und mit einem handelsüblichen Haushalts-Handmixgerät (Firma Krups) gut durchmischt. Anschließend wird das benetzte Polymer für 30 Minuten in einem Ofen auf eine Temperatur von 180°C erhitzt.

| Beispiel Bezeichnung. | Retention [g/g] | AAP [g/g] |
|---|---|---|
| 3a | 32,0 | 22,5 |
| 3b | 28,0 | 24,5 |
| 3c | 27,0 | 23,5 |

**Beispiel 4)**

Bei diesem Beispiel erfolgt die Oberflächenvemetzung nach der Zugabe von Cyclodextrin und Zeolith.

50 g der getrockneten, gemahlenen und auf 150-850 µm abgesiebten Polymeren aus Beispiel 2a-d) werden jeweils einzeln unter kräftigen Durchmischen mit einer Lösung aus Ethylencarbonat (EC) und Wasser in einem Kunststoffgefäß benetzt und mit einem handelsüblichen Haushalts-Handmixgerät (Firma Krups) gut durchmischt. Die Lösung enhält 0,25 g EC pro 1,8 g Wasser. Anschließend wird das benetzte Polymer für 30 Minuten in einem Ofen auf 170° C erhitzt.

| Beispiel Bezeichnung. | EA (CD) [%] | Retention [g/g] | AAP [g/g] |
|---|---|---|---|
| 4a | 10 | 27,2 | 22,2 |
| 4b | 38 | 26,4 | 21,8 |
| 4c | 56 | 28,6 | 21,5 |
| 4d | 57 | 29,0 | 20,3 |

**Vergleichsbeispiel 3a) (analog zu WO 91/12029)**

10 g Methylcellulose (Walocel VP-M 20678) werden mit 40 g Flavith® S108 (Degussa AG, $SiO_2/Al_2O_3$ Verhältnis ca. 500) und 190 g Wasser mit Hilfe eines Hochgeschwindigkeitsmixers dispergiert und anschließend in einem Labormischer mit 50 g eines handelsüblichen Superabsorbers (Favor® SXM 6860 der Firma Stockhausen) gemischt und in einem Wirbelschichttrockner bei 60°C und konstantem Luftstrom für 20 min getrocknet.

**Vergleichsbeispiel 3b) (analog zu WO 91/12029)**

0,25 g Methylcellulose (Walocel VP-M 20678) werden mit 1 g Flavith® S108 und 5 g Wasser mit Hilfe eines Hochgeschwindigkeitsmixers dispergiert und anschließend in einem Labormischer mit 50 g eines handelsüblichen Superabsorbers (Favor® SXM 6860 der Firma Stockhausen) gemischt und in einem Wirbelschichttrockner bei 60°C und konstantem Luftstrom für 20 min getrocknet.

| Bezeichnung | TB [g/g] | AAP 0,3 psi [g/g] | AAP 0,7 psi [g/g] |
|---|---|---|---|
| V3a | 20,5 | 16,2 | 9,0 |
| V3b | 29,0 | 27,5 | 18,4 |
| SXM 6860 | 31,0 | 31,0 | 24,0 |

Neben der drastisch verschlechterten Leistung insbesondere bei der Absorptionsfähigkeit unter Druck verglichen mit SXM 6860, zeigt sich die Instabilität des Komposit-Materials bereits durch eine starke Staubentwicklung bei Mischung oder Förderung des Materials.

**Vergleichsbeispiel 4)**

Bei diesem Beispiel erfolgt der Zusatz von Cyclodextrin und Zeolith nach der Oberflächenvernetzung.
50 g des aus Beispiel 3) erhaltenen Produktes werden mit den in der nachfolgenden Tabelle angegebenen Menge an Cyclodextrin und Zeolith (Flavith® S108, Fa. Degussa-Hüls) in Wasser suspendiert unter guter Durchmischung besprüht. Das Produkt wird im Trockenschrank bis auf einen Restwassergehalt von <4% getrocknet.

| Bezeichnung | Beta-CD [%] | Flavith S108 [%] |
|---|---|---|
| V4a | 0,5 | 2 |
| V4b | 0,5 | 1 |
| V4c | 2 | 2 |
| V4d | 1 | 1 |

**Beispiel 5)**

Das aus Vergleichsbeispiel 4) erhaltene Produkt wird gesiebt und die Fraktion mit einer Korngröße von 150-850 µm einem Kugelmühlenstabilitätstest unterzogen. Dabei wird das Produkt 6 Minuten bei 95 Upm in der Kugelmühle belastet. Das aus Beispiel 2d erhaltene Produkt wurde ebenfalls einem Kugelmühlenstabilitätstest unterzogen. Die Produkte wurden erneut gesiebt und die Fraktion mit einer Korngröße von <150 µm nach Prüfmethode 5 auf den Siliziumgehalt untersucht. Da der eingesetzte Zeolith eine Korngröße von deutlich unter 150 µm aufweist, läßt sich mit dieser Methode ermitteln wieviel Zeolith am Polymer mit superabsorbierenden Eigenschaften gebunden oder eingeschlossen ist. Für die Proben wurden folgende Mengen an Silizium bezogen auf die Gesamtmenge Trockensubstanz gefunden:

| Produkt aus Beispiel | $SiO_2$-Gehalt [%] | Theoretischer $SiO_2$-Gehalt* [%] |
|---|---|---|
| 2d | 0,7 | 0,75 |
| Vergleichsbeispiel V4a | 3,5 | 2 |
| Vergleichsbeispiel V4d | 5,8 | 2 |

* bezogen auf die Gesamtmenge an erfindungsgemäßem Polymer mit superabsorbierenden Eigenschaften.

Es ist klar zu erkennen, daß die erfindungsgemäßen Produkte in der Fraktion der Partikel <150 µm deutlich weniger Silizium aufweisen als ein nach dem Stand der Technik hergestelltes Produkt. Dies belegt, daß die Anbindung des Zeolithen an das Polymer bei den erfindungsgemäßen Produkten deutlich stärker ist. Durch mechanische Belastung des Materials lösen sich bei Produkten gemäß dem Stand der Technik große Mengen des Zeolithen, gekennzeichnet durch den hohen Siliziumdioxidgehalt im Feinstaub nach Kugelmühlenbelastung.

**Beispiel 6)**

Bei diesem Beispiel wurde die Verminderung der Dampfraumkonzentration geruchsbelästigender Verbindungen untersucht.
Bei der Messung der geruchsbelästigenden Stoffe wurde als Blindprobe gemäß Prüfvorschrift 2) ein Polymer ohne Cyclodextrin und ohne Zeolith untersucht und die gefundene Dampfraum-Konzentration der geruchsbelästigenden Substanz mit 100% gleichgesetzt. Anschließend wurden Cyclodextrin-und/oder Zeolithhaltige Proben untersucht und die Dampfraum-Konzentration der geruchsbelästigenden Substanz bestimmt. Die Angabe in der rechten Spalte errechnet sich nach: 100*(gefundene Menge Geruchssubstanz aus CD/Zeolithhaltigem Polymer/ gefundene Menge Geruchssubstanz aus CD/Zeolith-freiem Polymer).

**Dotierung mit Furfurylmercaptan:**

| **Polymer gemäß Beispiel** | Abnahme der Konz. Von Furfurylmercaptan im Dampfraum [%] |
|---|---|
| 4a | 62,4 |
| 4b | 63,0 |
| 4c | 71,0 |
| 4d | 75,3 |

Die erfindungsgemäßen absorptionsfähigen Polymerisate zeigen eine deutliche Aufnahme von geruchsbelästigenden Substanzen.

**Patentansprüche**

1. Absorptionsfähiges, vernetztes Polymerisat für Wasser oder wässrige Körperflüssigkeiten, auf Basis von gegebenenfalls teilneutralisierten, monoethylenisch ungesättigten Säuregruppen tragenden Monomeren, wobei das Polymerisat Cyclodextrin oder Cyclodextriderivate und siliziumreiche Zeolithe mit einem Siliziumoxid/Aluminiumoxidverhältnis von > 10 mindestens teilweise kovalent, ionisch gebunden oder darin eingeschlossen enthält

2. Polymerisat nach Anspruch 1, wobei dieses 0,01-50 Gew.%, Cyclodextrin oder Cyclodextrin derivate und mindestens 0,01-10 Gew.%, siliziumreiche Zeolithe enthält.

3. Polymerisat nach einem der Ansprüche 1 oder 2, wobei der extrahierbare Anteil des Cyclodextrins oder dessen Derivate maximal 85 % beträgt.

4. Polymerisat nach einem der Ansprüche 1 bis 3, wobei es mit 0,01 bis 30 Gew.%, bezogen auf das Polymerisat, einer Vernetzerkomponente beschichtet wurde, die mit mindestens zwei Carboxylgruppen in der Oberflächenschicht der Polymerpartikel reagiert und eine Vernetzung bewirkt.

5. Verfahren zur Herstellung von Polymerisaten nach einem der Ansprüche 1 bis 4, durch radikalisierte. Polymerisation einer wässrigen Lösung aus ethylenisch ungesättigten, Säuregruppen tragenden, gegebenenfalls teilneutralisierten Monomeren gegebenenfalls bis zu 30 Gew. % weiteren monoethylenisch ungesättigten Comonomeren, vernetzenden Monomeren, und gegebenenfalls bis zu 40Gew.% eines wasserlöslichen natürlichen oder synthetischen Polymeren nach dem Verfahren einer Lösungs- oder Suspensionspolymerisation zu einem Hydrogel, gegebenenfalls Isolierung, Zerkleinerung gefolgt von Trocknung, Mahlung/Siebung, wobei die Cyclodestrinkomponente und der siliziumreiche Zeolith mit einem Siliziumoxid/Aluminiumoxid-Verhältnis von > 10 spätestens während

einer Oberflächenvemetzung dem Polymerisat zugesetzt werden, vorzugsweise als Lösung oder in Suspension.

6. Verfahren zur Herstellung von Polymerisaten nach Anspruch 5, wobei das Hydrogel oberflächenvernetzt wird.

7. Verfahren nach einem der Ansprüche 5 oder 6, wobei der pH-Wert der Cyclodextrinlösung und/oder Suspension von Zeolith auf größer 8, bevorzugt größer 9 und ganz besonders bevorzugt > 11 eingestellt wird.

8. Verwendung der Polymerisate nach einem der Ansprüche 1 bis [7] 4 zur verbesserten Aufnähme von Gerüchen aus Körperflüssigkeiten, oder als Absorptionsmittel für wässrige Flüssigkeiten, vorzugsweise in Konstruktionen zur Aufnahme von Körperflüssigkeiten, in geschäumten und nicht geschäumten Flächengebilden, in Verpackungsmaterialien, bei der Pflanzenzucht und als Bodenverbesserungsmittel; oder als Trägersubstanz und/oder Stabilisator für Wirkstoffe, insbesondere für Düngemittel oder andere Wirkstoffe, die gegebenenfalls retardiert abgegeben werden.

9. Verwendung der Polymerisate nach einem der Ansprüche 1-4 in Hygieneartikeln.

**Claims**

1. An absorbent, crosslinked polymer for water or aqueous body fluids, based on optionally partially neutralized, monoethylenically unsaturated monomers bearing acid groups, wherein the polymer has cyclodextrin or cyclodextrin derivatives and zeolites high in silicon with a silicon oxide/aluminium oxide ratio of > 10 at least partially bound covalently, ionically bound thereto or incorporated therein.

2. The polymer according to claim 1, wherein it contains 0,01-50 wt.-% cyclodextrin or cyclodextrin derivatives and at least 0,01-10 wt.-% of zeolites high in silicon.

3. The polymer according to one of claims 1 or 2, wherein the extractable amount of cyclodextrin or derivatives thereof is 85 % at maximum.

4. The polymer according to one of claims 1 to 3, wherein it has been coated with 0,01-30 wt.-% relative to the polymer, of a crosslinker component which reacts with at least two corboxylic groups in the surface layer of the polymer particles, thereby effecting crosslinking.

5. A process for producing the polymers according to one of claims 1 to 4, by radical polymerization of an aqueous solution of ethylenically unsaturated, optionally partially neutralized monomers bearing acid groups, optionally up to 30 wt.-% of further monoethylenically unsaturated comonomers, crosslinking monomers, and optionally up to 40 wt.-% of a water-soluble natural or synthetic polymer according to the process of solution or suspension polymerization to form a hydrogel, optional isolation, crushing, followed by drying, milling/screening, wherein the cyclodextrin component and the zeolite high in silicon with a silicon oxide/aluminium oxide ratio of > 10 are added to the polymer during a surface cross-linking at the latest, preferably as a solution or in suspension.

6. The process for producing polymers according to claim 5, wherein the hydrogel is surface-crosslinked.

7. The process according to one of claims 5 or 6, wherein the pH value of the cyclodextrin solution and/or zeolite suspension is adjusted to > 8, preferably > 9, and more preferably > 11.

8. Use of the polymers according to one of claims 1 to 4 for improved absorption of odors from body fluids, or as an absorbent for aqueous liquids, preferably in constructions for absorbing body fluids, in foamed and non-foamed sheet materials, in packaging materials, in plant breeding, and as soil improver; or as a vehicle and/or stabilizer for active substances, particularly fertilizers or other active substances being released optionally in a delayed fashion.

9. Use of the polymers according to one of claims 1-4 in hygiene articles.

## Revendications

1. Polymérisat réticulé absorbant l'eau ou les sécrétions corporelles liquides, à base de monomères porteurs de radicaux acides monoéthyléniquement insaturés, éventuellement partiellement neutralisés, dans lequel le polymérisat contient de la cyclodextrine ou des dérivés de cyclodextrine et des zéolithes riches en silicium avec un rapport oxyde de silicium/oxyde d'aluminium supérieur à 10 au moins, liés ioniquement et de manière partiellement covalente ou enfermés dans celui-ci.

2. Polymérisat selon la revendication 1, contenant entre 0,01 et 50 pour cent en poids de cyclodextrine ou de dérivés de cyclodextrine et au moins entre 0,01 et 10 pour cent en poids de zéolithes riches en silicium.

3. Polymérisat selon l'une des revendication 1 ou 2, dans lequel la part extractible de cyclodextrine ou de ses dérivés est au maximum de 85 %.

4. Polymérisat selon l'une des revendications 1 à 3, recouvert de 0,01 à 30 % d'un agent de réticulation en poids par rapport au polymérisat, ledit composant réagissant avec au moins deux groupes carboxyle de la couche de surface de la particule de polymère et provoquant une réticulation.

5. Procédé de production de polymérisats selon l'une des revendications 1 à 4 consistant en une polymérisation radicalaire d'une solution aqueuse composée de monomères éthyléniquement insaturés, porteurs de radicaux acides, éventuellement partiellement neutralisés, avec éventuellement jusqu'à 30 pour cent en poids de comonomères supplémentaires monoéthyléniquement insaturés, de monomères réticulants et éventuellement jusqu'à 40 pour cent en poids d'un polymère hydrosoluble naturel ou synthétique, par un procédé de polymérisation en solution ou en suspension pour former un hydrogel, avec éventuellement une séparation, un broyage suivi d'un séchage, d'un broyage/tamisage, dans lequel les composants cyclodextrine et la zéolithe riche en silicium dont le rapport oxyde de silicium/oxyde d'aluminium est supérieur à 10 sont ajoutés au polymérisat au plus tard lors de la réticulation de surface, de préférence sous forme de solution ou de suspension.

6. Procédé de production de polymérisats selon la revendication 5, dans lequel l'hydrogel est réticulé en surface.

7. Procédé selon l'une des revendications 5 ou 6, dans lequel le pH de la solution de cyclodextrine et/ou de la suspension de zéolithe est ajusté de manière à être supérieur à 8, de préférence supérieur à 9 et de manière particulièrement préférée, supérieur à 11.

8. Utilisation des polymérisats selon l'une des revendications 1 à 4 pour une meilleure absorption des odeurs produites par les sécrétions corporelles ou comme moyen d'absorption de liquides aqueux, de préférence dans des agencements servant à l'absorption de sécrétions corporelles, dont les surfaces sont expansées et non expansées, dans des matériaux d'emballage, pour l'horticulture et comme moyen d'amélioration du sol ; ou bien comme substance vectrice et/ou stabilisatrice pour des matières actives, notamment pour des engrais ou d'autres matières actives devant éventuellement être libérées de manière prolongée.

9. Utilisation des polymérisats selon l'une des revendications 1 à 4 dans des articles d'hygiène.